Europäisches Patentamt

European Patent Office

Office européen des brevets

(19)

(11) Publication number : **0 440 409 A2**

# (12) EUROPEAN PATENT APPLICATION

(21) Application number : **91300647.4**

(51) Int. Cl.⁵ : **C12N 1/20, A61K 35/74**

(22) Date of filing : **29.01.91**

The microorganism(s) has (have) been deposited with Fermentation Research Institute under number FERM BP-3219.

(30) Priority : **29.01.90 JP 18595/90**

(43) Date of publication of application :
**07.08.91 Bulletin 91/32**

(84) Designated Contracting States :
**AT BE CH DE FR GB IT LI LU NL SE**

(71) Applicant : **KITASATO KENKYUSHO**
**9-1, Shirokane 5 chome Minato-ku**
**Tokyo-to (JP)**

(72) Inventor : **Tsurumizu, Takashi**
**176-2, Kuriyama Matsudo-shi**
**Chiba-ken (JP)**
Inventor : **Hashimoto, Takashi**
**1-21, Kami-ishihara Chofu-shi**
**Tokyo-to (JP)**
Inventor : **Sato, Makoto**
**2-34, Otsubo-jutaku, 232-1, Otusbo,**
**Hachiman-cho**
**Tokushima-shi Tokushima-ken (JP)**

(74) Representative : **Pett, Christopher Phineas et al**
**Frank B. Dehn & Co. European Patent Attorneys Imperial House 15-19 Kingsway**
**London WC2B 6UZ (GB)**

(54) **Non-cariogenic microorganisms and compositions containing them.**

(57)  A non-cariogenic microorganism is obtained by subculturing over many generations, a non-cariogenic strain of Human-type type S. mutans which is substabtially positive in the ability to decompose mannitol, melibiose and sorbitol and positive in the reactivity with anti-S. sanguis serum in the gel agglutination, until both said ability to decompose three sugars and said reactivity with anti S .sanguis convert into substantially negative.

The resultant microorganism may be used solely or in combination with at least one naturally-occurring Phase III strain of Human-type S. mutans for preventing or inhibiting dental caries of humans and mammals with good results.

EP 0 440 409 A2

## NON-CARIOGENIC MICROORGANISMS AND COMPOSITIONS CONTAINING THEM

The present invention relates to a process for the preparation of non-cariogenic microorganisms useful for preventing or inhibiting (hereinafter referred to as inhibiting) dental caries of humans and animals and to a non-cariogenic composition containing the living living cells obtained by the process of the present invention.

The term "non-cariogenic microorganism" used in this specification denotes a microorganism which is substantially deficient in cariogenic potential and which is capable of inhibiting the growth of cariogenic bacilli in the oral cavity of humans and animals. The non-cariogenic microorganism as defined above is substantially deficient in cariogenic potential and is capable of inhibiting the productivities of water-soluble and insoluble dextran-like polysaccharides (hereinafter referred to as DPS) and plaque.

Dental caries is a disease which occurs on the surface of teeth and progressively breaks down the structure of teeth and which is directly induced by the action of cariogenic bacilli in the oral floras. Among them, Streptococcus mutans is most important.

As is well known, S. mutans adheres onto the surfaces of teeth and decomposes sucrose to form dental plaque comprising insoluble DPS. The plaque is solid and adhesive and adheres to the surfaces of the teeth. S. mutans is believed to be most important by the reason that S. mutans resides in the plaque and produces lactic acid and acetic acid, although other cariogenic bacilli also form plaque.

British Patent 1,375,866 discloses that formation of dental plaque is a pre-requisite for the induction of dental caries and that glycosyltransferase (GT-ase) is an important factor in the development of dental caries.

It is also known that streptococci other than S. mutans, such as S. sanguis, S. mitis and S. salivarius, as well as edible lactic acid-producing bacilli form dental plaque and induce dental caries.

It has hitherto been proposed to inhibit dental caries by using the living cells of S. mutans JH140 (ATCC 31341) or JH145 (ATCC 31377), which are mutant strains capable of inhibiting cariogenic strains of S. mutans (U.S. Patent 4,133,875 to Hillman). These strains are obtained by culturing a streptomycin-resistant S. mutans BHT-2 in a special medium, followed by treating with a mutagen. But JH140 and JH145 did not give good results when used in practice because, for example, the following problems arose :-

Makoto Sato, one of the co-inventors of the present invention has classified S. mutans into Human I, Human II and Rat-types. Sato has reported as follows :-

1) 93.9 % of S. mutans of human origin were Human I type and the rest were Human II type ;
2) all strains of rat origin were Rat-type ;
3) no S. mutans was isolated from mice and guinea pigs ; and
4) all strains originating from monkeys and hamsters were Human I type.

Human I-, II- and Rat-types respectively correspond to serotypes "c, e and f", "d and g" and "a and b" according to the classification by Bratthal , and by Perch et al.
[J. Dental Health, Vol. 23, No.2, pp. 100-123, 1978, in Japanese version].

BHT-2, the parent strain of JH140 and JH145 is a Rat-type strain which has never been found in the oral cavity of humans, and Hillman does not disclose what will happen on administration of his mutant strains into the oral cavity of humans.

JP-A-164517/83 discloses a vaccine for inhibiting dental caries which comprises as active ingredient the living cells of S. mutans Attenuated Strain K-III (FERM-BP 316) which was obtained by treating a cariogenic strain of S. mutans isolated from a human oral cavity with a mutagen . This vaccine exhibits good non-cariogenicity, but has a similar disadvantage to that inherent to Hillman's strains in view of the fact that K-III is induced from cariogenic S. mutans strains of human origin.

European Patent No. 90617, discloses that :
(A) It is well known that S. mutans is highly mutative. We have classified S. mutans into the following three mutational phases disregarding the serotypes and biotypes of strains :-
(1) Phase I :
"Normal" phase widely distributed in the oral floras of humans and animals. The plaque formed is abundant, solid and adhesive. The productivity of DPS and lactic acid is high. The cariogenic potential is the highest of all 3 phases.
(2) Phase II :
Essentially unstable phase. There is a mutational variability between Phases I and II. The productivity of lactic acid and cariogenicity are weaker than those of Phase I.
(3) Phase III :
Obtainable by treating Phase I with a mutagenic agent. Various characteristics of this phase are entirely different from the characteristics of other phases. In the test tube, the productivity of lactic acid is low. The ability to produce insoluble and water-soluble DPS is substantially reduced.

It has been found that Phase III strains are capable of inhibiting the growth of Phase I strains in coexistence with them. Where a Phase III is introduced into a culture or into the the oral cavity of animals where the plaque has been formed by the action of Phase I strains, Phase III intrudes into the plaque to reside there so that Phase I strains are outwardly destroyed and gradually disappear owing to the loss of residence. Phase III strains also die owing to the loss of their support medium or residence and their inability to form new plaque.

In the case where Phase I strains are introduced into a test tube or oral cavity already occupied by Phase III, Phase I strains die. Phase III strains are capable of completely inhibiting the growth of cariogenic strains of S. mutans and edible lactic acid-producing bacilli.

(B) We have found that Phase-III like strains of Human type S. mutans (hereinafter referred to as naturally-occurring Phase III) are living in the oral cavity of humans. These strains exhibit substantially similar characteristics to the characteristics of Phase III strains which we have induced artificially (hereinafter referred to as artificial Phase III) from cariogenic strains of S. mutans.

Naturally-occurring Phase III strains have been isolated from the oral cavity of humans and purely cultured.

Table 1 indicates the decomposition of sugars by naturally-occurring Phase III strains which we have isolated from the oral cavity of humans. In this table, the following abbreviations are used :-

```
Ag: agglutination titre,

Ma: mannitol,   So: sorbitol,     Ra: raffinose,

Sa: salicin,    Ce: cellobiose,   Me: mellibiose,

La: lactose,    Su: sucrose,      Es: esculin
```

The corresponding values of S. mutans NCTC 10449, a typical cariogenic S. mutans of wild type are also shown here for comparison purpose.

## TABLE 1

| Strain (TMD-NC) | FERM BP. | Ag | Ma | So | Ra | Sa | Ce | Me | La | Su | Es |
|---|---|---|---|---|---|---|---|---|---|---|---|
| Inoue | 3058 | 256 | + | + | + | + | + | + | + | + | + |
| T. Komatsu | 3059 | 2048 | + | + | + | + | + | + | + | + | + |
| K. Komatsu | 3060 | 1024 | + | + | + | − | + | + | + | + | + |
| Kawabe | 3061 | 1024 | + | + | + | − | + | + | + | + | − |
| Nakayama | 3062 | 1024 | + | + | + | − | + | + | + | + | − |
| Kume* | 319 | 1024 | + | + | + | − | + | − | + | + | + |
| 26P | 3063 | 256 | + | + | + | + | + | + | + | + | + |
| 49 | 318 | 256 | + | + | + | + | + | + | + | + | − |
| 57-4 | 3064 | 1024 | + | + | + | + | + | + | + | + | + |
| NCTC10449** | | 2048 | + | + | − | − | + | + | + | + | + |

Notes:-

\* All strains are Human I type (serotype c) except Kume (Human II type, serotype d).

\*\* Other decompositions are the same as those of NCT 10449 (Phase I), otherwise specified.

The characteristics of naturally-occurring Phase III are as follows :-

I. Morphology :

Streptococcus mutans, 1μ X 1-2μ, Gram-positive.

II. Growth on various media [37 °C , 24 hours, specified pH] :

(1) TYC agar plate medium (Stoppelaar et al, Oral Biol., 12, 1199-1201, 1976) :

Insoluble and water-soluble DPS are not produced.

Colonies are round, flat, white, opaque, bright and large.

(2) Todd Hewitt broth agar plate medium (Baltimore Biological Laboratories, Inc., U.S.A., hereinafter referred to as BBL) :

Colonies are round, flat, opaque, bright, somewhat mucoid and large.

(3) Mitis-salivarius agar plate medium (Difco., U.S.A.) :

DPS are not produced. Colonies are round, pale whitish, somewhat mucoid and large.

(4) Brain Heart Infusion agar plate medium (BBL) :

Colonies are round, somewhat whitish, opaque and large.

(5) Tryptocase Soy Broth (BBL) :

Growing from the lower layer of the medium.

(6) Todd Heuwitt Broth (BBL) :

Growing from the lower layer of the medium.

III. Physiological characteristics :

(1) Productivity of wire plaque (showing the adhering ability by insoluble DPS) : negative.

(2) Agglutination of cells by DPS : negative,

(3) Formation of pigment : negative.

(4) Growth range : pH 5-8, temperature 10-30 °C.

(5) Decomposition of sugars : as shown in Table 1.

IV. Miscellaneous :

Hydrolysis of esculin : positive.

Hemolysis (sheep) : from positive to negative.

Glucosyltransferase activity : substantially deficient.

Productivity of lactic acid : very weak

Ability to infect to humans and animals : very weak

Cariogenicity : substantially deficient.

V. Immunological characteristics :

Serotype : Human I or Human II type.

Naturally-occuring Phase III strains represent non-cariogenic microorganisms as hereinbefore defined, and are capable of effectively inhibiting the growth of cariogenic streptococci and edible lactic acid-producing bacilli in vitro and in vivo.

Naturally-occurring Phase III strains may be cultured in a conventional manner as in the case of known S. mutans. Thus, the culturing may be effected aerobically, although the culturing under anaerobic conditions is preferred. Although both organic and synthetic media may be used, liquid media is preferred for mass propagation.

Various media suitable for culturing streptococci may be used, for example, at a pH of from 5.6-9.0 (preferably about 7.0) and at a temperature of 23-39°C (preferably 37°C). After completion of culturing, for example, for 12-72 hours, the cultured broth contains, for example, about $10^8$ cells/ml.

(C) It is possible to completely inhibit dental caries induced by cariogenic streptococci and edible lactic acid-producing bacilli by using a composition which comprises an effective amount of the living cells of naturally-occurring Phase III strains, in association with a carrier or excipient suitable for oral administration.

Although it is possible to administer the cultured liquor to the oral cavity of humans without any after-processing, it is preferred to separate the living cells from the cultured broth and suspend the cells in a suitable carrier. Alternatively the isolated cell may be freeze-dried for preservation. In use, the cells are suspended in a suitable carrier. The methods for freeze-drying and preservation are well known in the art.

Because their ability to adhere is weak, in addition to the loss of plaque-forming ability, it is preferred to administer a large amount of the strain into the oral cavity of humans. A liquid composition may preferably contain, for example, about $10^5$-$10^8$ living cells per ml. For example, in the case where a non-cariogenic composition containing about $10^7$-$10^9$ living cells of edible lactic acid-producing bacilli and about $10^6$ living cells of naturally-occurring Phase III strains per ml was administered every day at a dose of 50-100 ml, it has been observed that cariogenic strains of streptococci and edible lactic acid-producing bacilli in the oral cavity disappeared in about 1-2 weeks after the beginning of administration.

The present invention is based upon the discovery that, by subculturing S. mutans TMD-NC 49 (FERM-BP 318), one of the naturally-occurring Phase III strains over extended generations in media, there was obtained a non-cariogenic microorganism which exhibits certain different characteristics which make it preferred over the parent strain while retaining the excellent dental caries-inhibiting activity of the parent strain.

The present invention is directed to provide a process for the preparation of non-cariogenic micro-organisms and a non-cariogenic composition containing such microorganisms.

According to one feature of the present invention, there is provided a process for the preparation of non-cariogenic microorganisms, which comprises subculturing a strain of Streptococcus mutans as a parent strain in culture media until the ability to decompose mannitol, sorbitol and melibiose and the reactivity with anti-Streptococcus sanguis serum convert from substantially positive to substantially negative, and recovering the resultant microorganism therefrom, said parent strain being (a) isolated from the oral cavity of humans, (b) substantially deficient in cariogenic potential and capable of inhibiting the growth of cariogenic streptococci and edible lactic acid-producing bacilli in the oral cavity of humans and mammals, and (c) capable of converting its ability to decompose mannitol, sorbitol and melibiose and its reactivity with anti-Streptococcus sanguis serum in the gel agglutination reaction from substantially positive to substantially negative by subculturing in the media.

Another aspect of the present invention provides a non-cariogenic composition for preventing or inhibiting dental caries of humans and mammals, induced by cariogenic strains of the genus Streptococcus and edible lactic acid-producing bacilli, which comprises as active ingredient the living cell of a microorganism prepared by the process of the present invention, in association with a carrier or excipient suitable for oral administration.

The present invention may be more clearly understood from the following description.

A microorganism was obtained by subculturing S. mutans TMD-NC 49 (FERM-BP 318), one of the naturally occurring Phase III strains, in culture media over many generations. This has been obtained in the form of a pure culture. This has characteristics different from those of the parent strain as set forth. The resultant microorganism has been named by us as Streptococcus sp, 6480 and filed with the Fermentation Research Institute (FRI, the depositary institution of the Japanese government) located at 1-3, Higashi 1-chome, Tsukuba-shi, Ibarakiken, Japan on 19th December 1989, the deposition number of this strain being FERM-P 11170. This was transferred to the international depository institution on 28th December 1990 under the Budapest Treaty as FERM-BP 3219.

Non-cariogenicity and other biological characteristics of Streptococcus. sp. 6480 are substantially the same as those of the parent strain S, mutans TMD-NC 49.

The following abbreviations are used in the following tables :-

A - S. mutans NCTC 10449 (ATCC 25175), a representative wild type cariogenic strain (Phase I).

B - S. mutans TMD-NC 49, parent strain.

C - Streptococcus sp. 6480 (FERM-BP 3219).

D - S. mutans Attenuated Strain K-III (FERM-BP 316), an artificially induced Phase III strain.

STR-Streptococcus ATCC 15910.

## TABLE 2

|  | A | B | C | D |
|---|---|---|---|---|
| Formation of DPS | + | ± | − | ± |
| Decomposition of sugars: |  |  |  |  |
| mannitol | + | + | − | + |
| sorbitol | + | + | − | + |
| melibiose | + | + | − | + |
| Formation of acids(mM): |  |  |  |  |
| lactic acid | 2.27 | 2.21 | 2,16 | 2.51 |
| acetic acid | 0.18 | 0 | 0 | 0.08 |

Table 3 indicates the results of gel agglutination reaction with anti-oral streptococci antisera, by the use of polysaccharide antigens extracted from the selected streptococci. The results have been obtained by the method of Kohley et al. [Applied Microbiol., 28 : 836. 1974).

## TABLE 3

| Sample | A | B | C | D |
|---|---|---|---|---|
| SM NCTC10449 | + + | ± | ± | + |
| Sanguis | + + | + | - | + |
| Mitis | + + | - | - | ± |
| STR | + | + + | + + | + |

For example, with reference to Bergy's Manual of Determinative Bacteriology, page 1055 (1989), strains of S. mutans are capable of decomposing mannitol, sorbitol and melibiose. Both naturally-occurring Phase III and S. mutans Attenuated Strain K-III artificially induced from wild-type cariogenic strains are capable of decomposing mannitol, sorbitol and melibiose, while Streptococcus sp. 6480 is substantially incapable of decomposing mannitol, sorbitol and melibiose.

The parent strain S. mutans TMD-NC 49 exhibits a substantially positive reactivity with anti-S.sanguis antiserum in the gel agglutination.

The new strain may be obtained by subculturing the parent strain over many generations. For this purpose, various media conventionally used for culturing streptococci may be used. For mass propagation, it is preferred to use liquid media under anaerobic conditions.

The media which we have used for subculturing are exemplified as follows :-

(1) Todd Hewitt Broth (commercially available from Baltimore Biological Research Laboratories, U.S.A. hereinafter referred to as BBL) and

(2) Skimmed milk medium (skimmed milk 5 % ; yeast extract 0.5 %).

Usually culturing may be effected at a pH of 5.6-9.0 (for example, 7.0) and at a temperature of 23-39°C (for example, 37°C) for a period of 18-72 hours under anaerobic conditions.

It has been observed that the ability to decompose above-mentioned 3 sugars changes from substantially positive to substantially negative via pseudo-positive by subculturing over many generations. No deleterious interference between two microorganisms has been observed during subculturing. Although the times of subculturing may vary with differing conditions such as, for example, the media used, the subculturing may be effected, for example, more than 80-200 times.

For example, after completion of the first culturing, a small amount of the cultured broth is transferred to a test agar medium for culturing under the same conditions. The resultant colonies may be selected with reference to the above-mentioned characteristics and the selected colony may be used for subsequent subculturing.

By the test culturing combined with the following selection (hereinafter referred to as cloning), it is possible to obtain the desired strain by subculturing, but it is not always required to carry out cloning after every subculturing. If desired, it is possible to use different media for subculturing.

Use of sp. 6480 and non-cariogenic composition :

Strain sp. 6480 exhibits substantially the same non-cariogenicity as that of the parent strain and may be used in the same manner as that applied to the parent strain for effectively inhibiting dental caries of humans and mammals, induced by cariogenic streptococci and edible lactic acid-producing bacilli.

It is possible to replace the living cells of the new strain partly by the living cells of at least one member of naturally-occurring Phase III.

In particular at the earlier stage of subculturing, both the new strain and its parent strain co-exist in a same medium. Thus it is possible, if desired, to discontinue the subculturing optionally and use the cultured broth for inhibiting dental caries in disregard of the mixing ratio of two microorganisms.

Although it is possible to use the cultured broth without any after-treatment for administration into the oral cavity of humans and mammals, it is preferred, for example, to separate the living cells from the cultured broth in conventional manner by centrifugation (8000 r.p.m.) and suspend them in a suitable buffer solution such as a phosphate-buffered solution (pH 6.8-7.0) for freeze-dried. The freeze-dried cells may be preserved over an extended period of time.

The non-cariogenic composition may be in the form of a solid, liquid or semi-solid (like wheat-gluten). Suitable liquid carriers are exemplified by water, syrup and mammal's milk. Mixed drinks such as yogurts which contain living cells of edible lactic acid-producing bacilli are especially advantageous with respect to the inhibition of dental caries induced by cariogenic streptococci and edible lactic acid-producing bacilli.

It has been observed that, for example, in the case where a liquid composition containing $10^5$-$10^8$ living cells of Streptococcus sp. 6480 either solely or in combination with at least one naturally-occurring Phase III strains was administered to humans once daily after meal at a dose of 50-100 ml, it has been observed that the cariogenic strains of S. mutans gradually decreased in number about 1-2 weeks after the beginning of the administration.

In the following Experiment No. 2, showing the inhibition of dental caries of humans infected with cariogenic S. mutans, the results obtained by using cariogenic Human I type S. mutans (serotype "c") for infection were not significantly different from the corresponding results obtained by using cariogenic Human II-type S. mutans (serotype "d") for infection because there is no direct relationship between the ability to inhibit the plaque formation and the serotype of S. mutans.

Dental caries of pet mammals such as house dogs and house cats, induced by infection of S. mutans of human origin has recently been increasing. We have examined many pet dogs to note that the infection ratio of Human-type S. mutans is about 60 %. It has been observed that such dental caries may effectively be prevented or inhibited by the use of sp. 6480 solely or in combination with at least one of non-cariogenic strains of naturally-occurring Phase III S. mutans.

EXAMPLES :

The following non-limiting examples and experiments illustrate the invention. Hereinafter, culturing was effected at a temperature of 37 °C anaerobically by the use of Gaspack System (commercial product of BBL).

Hereinafter strains 49 and sp. 6480 respectively denote S. mutans TMD-NC 49 (FERM-BP 318) and Streptococcus sp. 6480 (FERM-P 11170). S.mutans ATCC 25175 (NCTC 10449) was used to induce dental caries.

Example 1 :

Preparation of strain sp. 6480 :

S. mutans 49 was cultured for 18 hours by using a skimmed milk medium (5 ml ; as set forth). The cultured broth (one platinum spoonful) was transferred to TYC agar medium (15 ml ; commercial product of Kyokuto Seiyaku K.K, Japan) for further culturing for 46 hours.

With reference to the decomposition of sugars, the resultant colonies were selected. The selected colony was subcultured over more than 150 generations in the same manner as that described above. The cultured broth was examined and centrifuged (8000 r.p.m./10 min.) in conventional manner to obtain the desired Streptococcus sp. 6480.

Example 2 :

Stick :

By mixing living cells of strain sp. 6480 in an amount of $10^6$ CFU, as obtained in Example 1, with maltose (0.75 g) and lactose (0.25 g) in conventional manner to obtain a stick.

Example 3 :

Tablet :

1.5 g of maltose, 0.5 g of lactose and 0.1 g of sucrose fatty acid ester were used to prepare a tablet containing $10^6$ CFU of living cells of strain sp. 6480, obtained in Example 1 in conventional manner,

Experiment 1 :

Inhibition of dental caries (animals) :

As test animals, Golden hamsters (male and female ; 21 days after birth) were used to carry out a test for 40 days. A cariogenic strain (S. mutans ATCC 27175=NCTC 10449) was infected to the animals of the first and second groups respectively consisting of 10 and 9 animals, by oral administration. After this, $10^6$ living cells of strain 49 and strain sp. 6480 were respectively given to the animals of the first and second groups. Strain sp. 6480 was infected orally into the third group consisting of 11 animals (uninfected), Then animals of each group were fed with Diet 2000 (cariogenic diet, commercial product of Funabashi Nojo, Japan) ad libitum.

The 4th group (the first control group) consisting of 9 animals was uninfected with cariogenic S. mutans and untreated. The 5th group (the second control group) consisting of 11 animals was uninfected with cariogenic S. mutans and fed with the cariogenic diet.

$10^6$ living cells of strain 49 or strain 6480 was orally given to the test animals once every day.

In Table 4, the following abbreviations are used :-

A : Increased body weight (g),

B : Carious score,

C1 : Infection ratio (%),

C2 : Number of infected teeth.

This table indicates that both strain sp. 6480 and its parent strain 49 inhibited effectively dental caries induced by S. mutans and that no significant difference was observed between two strains.

### TABLE 4

| Group | A | B | C1 | C2 |
|-------|-------|------------|-----|-------------|
| 1 | 105.4 | 12.8± 0.63 | 50 | 1.10 ± 1.28 |
| 2 | 173.8 | 11.0± 0.86 | 44 | 0.77 ± 1.30 |
| 3 | 160.4 | 11.9± 0.4 | 18 | 0.27 ± 0.64 |
| 4 | 184.0 | 12.7± 1.39 | 100 | 5.22 ± 3.80 |
| 5 | 157.1 | 11.7± 0.78 | 9 | 0.18 ± 0.60 |

### Experiment 2 :

### Inhibition of dental caries (humans) :

16 adult humans (male and female ; 20-28 years old) infected with Human I type (serotype "c") or Human II type (serotype "d") S. mutans, were tested.

To each member, one tablet described in Example 3 was orally given once daily before bedtime. In the oral cavity the tablet was dissolved and kept for a period of more than 15 minutes. The test period was 40 days.

The following Table 5 indicates the indexes of the adherence of dental plaque before and after administration. Table 6 indicates the decrease of the concentration of S. mutans in the oral floras by administration.

TABLE 5 (Adherence of dental plaque)

[No--Host No.; Sero--Serorotype of S. mutans
B--Before administration; D--Days after the beginning
of administration; Unit-plaque score]

| No. | Sero | B | D ( | 6 | 20 | 34 ) |
|-----|------|-----|-----|-------|-------|-------|
| 1 | c | 4.2 | | − 0.9 | − 0.9 | − 0.9 |
| 2 | c | 3.8 | | − 1.0 | − 1.8 | − 1.0 |
| 3 | c | 2.0 | | − 0.5 | − 0.2 | ± 0 |
| 4 | c | 2.7 | | ± 0 | − 0.5 | − 0.5 |
| 5 | c | 3.3 | | − 1.0 | − 0.8 | − 1.5 |
| 6 | c | 4.0 | | − 0.5 | − 1.0 | − 1.2 |
| 7 | c | 3.2 | | − 1.4 | − 1.0 | − 1.0 |
| 8 | c | 3.2 | | − 0.2 | − 0.4 | − 1.2 |
| 9 | c. | 1.5 | | + 0.3 | − 0.5 | − 0.5 |
| 10 | c | 3.2 | | − 1.5 | − 0.9 | − 1.2 |
| 11 | c | 1.5 | | ± 0 | ± 0 | − 0.2 |
| 12 | c | 3.2 | | − 1.2 | − | + 0.1 |
| 13 | c | 2.0 | | + 0.5 | − 0.3 | − 0.3 |
| 14 | d | 2.7 | | + 0.1 | − 0.5 | − 0.5 |
| 15 | c | 2.3 | | ± 0 | − 0.3 | − 0.6 |
| 16 | d | 2.2 | | − 0.2 | − 0.9 | ± 0 |
| Average | | − | | − 0.5 | − 0.7 | − 0.7 |

## TABLE 6 (Number of S. mutans)

[No—Host No.; Sero—Serorotype of S. mutans;
B—Before administration; D1—Days after the beginning
of administration; D2—Days after completion of the
administration; W.A.—Weighted average index]

| No. | Sero | B | D1 | | | D2 | |
|-----|------|---|-----|-----|-----|-----|-----|
| | | | 6 | 20 | 34 | 3 | 21 |
| 1 | c | 448 | 14.8 | 2.1 | 0 | 0 | 7.7 |
| 2 | c | 820 | 22 | 0 | 5 | 0 | 39 |
| 3 | c | 282 | 0.3 | 0 | 0 | 0 | 91 |
| 4 | c | 22 | 0 | 0 | 2 | 0 | 37 |
| 5 | c | 39 | 0 | 0 | 0 | 0 | 4.4 |
| 6 | c | 33 | 0 | 0 | 0 | 0 | 4.2 |
| 7 | c | 75 | 0 | 0 | 0 | 0 | 134 |
| 8 | c | 780 | 4.7 | 0 | 1.1 | 6.3. | 127 |
| 9 | c | 51 | 0.1 | 0 | 0 | 0 | 21.4 |
| 10 | c | 2.9 | 0.9 | 0.4 | 0 | 0 | 11 |
| 11 | c | 150 | 1.5 | 0 | 0 | 0 | 4 |
| 12 | c | 2.7 | 0 | 0 | 0 | 0.2 | 3.5 |
| 13 | c | 2.8 | 0.2 | 0.1 | 0 | 0 | 22 |
| 14 | d | 8.4 | 0 | 0.2 | 7 | 0 | 21 |
| 15 | c | 24 | 0 | 0.1 | 0 | 1.3 | 0 |
| 16 | d | 1.7 | 0.2 | 0.6 | 0 | 0 | – |
| Average | | 171.23 | 2.79 | 0.22 | 0.94 | 0.49 | 33.6 |
| W.A. | | 274.03 | 6.34 | 0.53 | 2.08 | 1.58 | 43.8 |

Experiment 3 :

Inhibition of dental caries (house dogs) :

69 house dogs (male and female) were used as test animals.On each occasion, dental plaque was collected from the molars and put into an aqueous solution of 3% peptone containing glycerol (10%), followed by freeze-drying. Each sample was dissolved by heating and put on TYC agar plate medium (commercial product of Kyokuto Seiyaku K.K, Japan) for culturing at a temperature of 37°C for 48 hours under anaerobic conditions. From the resultant colonies, microorganisms were collected and their biological and serological characterisitics were assayed in conventional manner. As a result, the presence of the following strains of S. mutans were found in the plaque collected from 37 house dogs (53.6 %) :-

Serotype c —15 (40.5 %), Serotype d --- 4 (10.8 %),
Undetectable—18 (49 %)

To each of 10 host was orally administered the preparate described in Example 2 (one pack) once daily after feeding. On the morning of the next day, the number of S. mutans in the saliva was counted. The results are shown in the following table, which indicate that the number of S. mutans (unit X 10^4 cells/ml) in the oral floras decreased gradually about 2 weeks after the beginning of administration. The results are shown in Table 7.

In this table, the following abbreviations are used :No.--Host dog ; Sero--Serotype of S. mutans ; B--Before administration ; A--After the beginning of administration ; * --undetectable.

## TABLE 7 (House dogs)

| No. | Sero | B | A (weeks) 2 | 4 |
|---|---|---|---|---|
| 1 | c | 150 | 11.1 | 0 |
| 2 | c | 40 | 0 | 0 |
| 3 | * | 13 | 0 | 0 |
| 4 | d | 6 | 0 | 0 |
| 5 | c | 29 | 0 | 0 |
| 6 | c | 211 | 0 | 0 |
| 7 | c | 8.6 | 1.5 | 0 |
| 8 | * | 75 | 0 | 0 |
| 9 | * | 313 | 13 | 0 |
| 10 | c | 8 | 0 | 0 |

## Claims

1) A process for the preparation of non-cariogenic microorganisms, which comprises subculturing a parent strain of Streptococcus mutans isolated from the oral cavity of humans in culture media, said parent strain further being substantially deficient in cariogenic potential, capable of inhibiting the growth of cariogenic streptococci and edible lactic acid-producing bacilli in the oral cavity of humans and mammals and capable of decomposing mannitol, sorbitol and melibiose and having a positive reactivity with anti-Streptococcus sanguis serum in the gel agglutination reaction, until said ability to decompose mannitol, sorbitol and melibiose and said reactivity with anti-Streptococcus sanguis serum convert from substantially positive to substantially negative, while said deficiency in cariogenic potential and growth-inhibiting capability remain, and recovering the resultant microorganism therefrom.

2) A process according to claim 1, wherein said parent is Streptococcus mutans TMD-NC 49 (FERM-BP 318).

3) A process according to claim 1 or 2, in which said resultant strain is Streptococcus sp. 6480 (FERM-BP 3219).

4) A non-cariogenic composition for preventing or inhibiting dental caries of humans and mammals, induced by cariogenic strains of streptococci and edible lactic acid-producing bacilli, comprising as active ingredient the living cell of a microorganism obtained by the process as claimed in claim 1, in association with a carrier or excipient suitable for oral administration.

5) A non-cariogenic composition according to claim 4, comprising as active ingredient, the living cells of the microorganism obtained by the process of claim 1 and the living cells of at least one strain of Streptococcus mutans which is isolated from the oral cavity of humans, said isolated strain being substantially deficient in cariogenic potential and capable of inhibiting the growth of cariogenic streptococci and edible lactic acid-producing bacilli in the oral cavity of humans and mammals.

6) A non-cariogenic composition for preventing or inhibiting dental caries in humans and mammals which comprises as active ingredient living cells of Streptococcus sp. 6480 (FERM-BP 3219) in association with a carrier or excipient suitable for oral administration.

7) A composition according to any of claims 4-6, further comprising as active ingredient the living cells of at least one strain of Streptococcus mutans isolated from the oral cavity of humans in culture media, said strain of Streptococcus mutans being substantially deficient in cariogenic potential, capable of inhibiting the growth of cariogenic streptococci and edible lactic acid-producing bacilli in the oral cavity of humans and mammals and capable of decomposing mannitol,sorbitol and melibiose and having a positive reactivity with anti-Streptococcus sanguis serum in the gel agglutination reaction.

8) A composition according to claim 5 or claim 7, in which said Streptococcus mutans is Streptococcus mutans TMD-NC 49 (FERM-BP 318).

9) A composition according to any of claims 4-8, in which the concentration of the living cells of Streptococcus sp. 6480 is from $10^5$ to $10^8$ cells on the basis of one ml of the carrier.

10) The microorganism Streptococcus sp. 6480 (FERM-BP 3219).